# EUROPÄISCHE PATENTSCHRIFT

(11) **EP 1 881 972 B1**
(45) Veröffentlichungstag und Bekanntmachung des Hinweises auf die Patenterteilung: **28.08.2013**
(21) Anmeldenummer: 06742787.2
(22) Anmeldetag: 04.05.2006
(51) Int. Cl.: C07D 317/40

(54) **Verfahren zur Herstellung von Vinylencarbonat**
Process for producing vinylene carbonate
Procédé de préparation de carbonate de vinylène

(30) Priorität: 12.05.2005 DE 102005021968
(43) Veröffentlichungstag der Anmeldung: 30.01.2008
(73) Patentinhaber: Saltigo GmbH, 40764 Langenfeld (DE)
(72) Erfinder: LANGER, Reinhard, 47918 Tönisvorst (DE); BECKMANN, Anke, 51067 Köln (DE); WAGNER, Paul, 40597 Düsseldorf (DE); GRZINIA, Heinrich, 41812 Erkelenz (DE); SCHNEIDER, Marielouise, Dr., 51375 Leverkusen (DE); NOTHEIS, Ulrich, 41539 Dormagen (DE); RODEFELD, Lars, 51375 Leverkusen (DE); MÜLLER, Nikolaus, 40789 Monheim (DE)
(74) Vertreter: Pettrich, Klaus-Günter
(86) Internationale Anmeldenummer: PCT/EP2006/004157
(87) Internationale Veröffentlichungsnummer: WO 2006/119911

(56) Entgegenhaltungen:
- DE-A1- 19 955 944

## Beschreibung

Die vorliegende Erfindung betrifft die technische Herstellung von Vinylencarbonat (VC) durch Abspaltung von Chlorwasserstoff aus Chlorethylenglykolcarbonat (CGC) in flüssiger Phase.

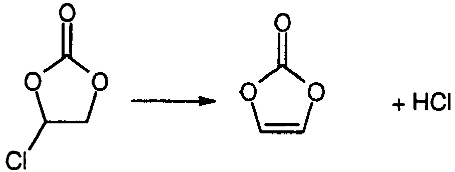

Vinylencarbonat ist ein wichtiges Zwischenprodukt für die Herstellung von Chemikalien, Pharmaprodukten, Pflanzenschutzmitteln und im Besonderen für Polymere, Lacke und Batterieelektrotyte.

Vinylencarbonat wird nach bekannter Methodik durch Abspalten von Chlorwasserstoff aus Chlorethylenglykolcarbonat mittels tertiärer Amine, insbesondere Triethylamin hergestellt.

Das Chlorethylenglykolcarbonat gewinnt man durch radikalische Chlorierung von Ethylenglykolcarbonat mittels Chlor oder Sulfurylchlorid.

Erstmals veröffentlicht wurde diese Synthese 1953 von Newman und Addor (JACS, 1953, S. 1263; JACS 1955, S. 3789).

Ethylenglykolcarbonat (GC) wurde mittels ultraviolettem Licht bei 60-70°C in Substanz photo-chloriert und das entstandene CGC durch Vakuumdestillation gereinigt.

VC erhielten Newman und Addor durch Eliminierung mittel Triethylamin in siedendem Ether (Kp. 35°C), wobei das Gemisch über Nacht erhitzt wurde.

Die Isolierung erfolgte durch Abfiltrieren des Triethylammoniumchlorides und anschließende Destillation, die mit 59%iger Ausbeute ein rohes VC lieferte, welches durch weitere Destillation gereinigt werden musste.

JP 2000/026449 beschreibt die Eliminierung in hochsiedenden Lösungsmitteln (Sdp. 170-300°C), explizit wird mit Triethylamin in Dibutylcarbonat 20 Stunden bei 50°C umgesetzt.

Nach dem Abfiltrieren des Ammoniumchlorides und dem Abdestillieren von überschüssigem Triethylamin wird rohes VC durch einfache Destillation isoliert. Um Spuren von Aminen zu entfernen wird das VC über eine Silicagelsäule gegeben. Abschließend wird eine Feindestillation durchgeführt. Der Chlorgehalt des so gewonnenen VC wird mit 29 ppm angegeben, während Vergleichsproben >3000 ppm enthalten. Die Ausbeute beträgt 56 %.

DE-A 19955944 beschreibt die Eliminierung in GC als Lösungsmittel (Sdp. 243-244°C). CGC wird in GC vorgelegt und in 1,5 Stunden durch Zugabe von Triethylamin bei 60°C umgesetzt. Nach Abdestillieren von überschüssigem Triethylamin bei 40°C und Verdampfen über einen Dünnschichter bei 100°C wird mit 73 %iger Ausbeute ein farbloses Gemisch aus VC und GC gewonnen. Über die Reinheit werden keine Angaben gemacht.

Die Umsetzungen von CGC in flüssiger Phase leiden alle unter der Zersetzlichkeit des VC, welche in DE-A 199 55 944 A1 explizit erwähnt wird. Oberhalb von 60°C zersetzt es sich demnach in Stunden und oberhalb von 80°C in Minuten. Die dabei entstehenden Polymere erschweren das Abtrennen der Salze und die exotherme Zersetzung macht das scale up solcher Prozesse problematisch.

Besonders schwierig ist, dass sowohl die Eliminierungsreaktion von CGC mit Triethylamin zu VC und Ammoniumchlorid als auch die Zersetzung des VC mit steigender Temperatur deutlich schneller werden.

Wünschenswert wäre ein Verfahren, das bei niedrigen Temperaturen höhere Umsätze und Selektivitäten liefert und eine leichte Isolierung des VC und des Ammoniumsalzes ermöglicht, nicht zuletzt durch gute Filtrierbarkeit des Salzes und gute Rührbarkeit des Reaktionsgemisches.

Gegenstand der Erfindung ist daher die Bereitstellung eines Verfahrens zur Herstellung von Vinylencarbonat, bei dem sich das VC in erhöhter Ausbeute bei besserer Isolierung des VC aus den Reaktionsgemisch herstellen lässt.

Überraschenderweise wurde gefunden, dass die gesuchten Verfahrenseigenschaften erzielt werden, wenn man ohne Lösungsmittel bzw. mit sehr wenig Lösungsmittel arbeitet, d.h. Vinylencarbonat selbst als Lösungsmittel dient.

Gegenstand der Erfindung ist ein Verfahren zur Herstellung von Vinylencarbonat durch Abspaltung von Chlorwasserstoff aus Chlorethylenglykolcarbonat (CGC) mit tertiären Aminen, dadurch gekennzeichnet, dass die Reaktion zwischen 30 und 60°C, in Vinylencarbonat als Reaktionsmedium, welches gegebenenfalls noch bis zu 50 Gew.-% weiteres Lösungsmittel, bezogen auf das Reaktionsmedium, enthält, durchführt.

Die Menge an inertem Lösungsmittel im Reaktionsgemisch liegt unterhalb 10 Gew.-%, bevorzugt unterhalb 3 Gew.-%, besonders bevorzugt unterhalb 1 Gew.-% liegt, ganz besonders bevorzugt wird lösungsmittelfrei gearbeitet. Die Gew.-% Angaben beziehen sich dabei auf das Reaktionsmedium. Als inerte Lösungsmittel sind z.B. aliphatische und aromatische Kohlenwasserstoffe aus der Gruppe der C₁-C₁₀-Alkane oder Cycloalkane sowie Benzol, das gegebenenfalls mehrfach mit C₁-C₁₀-Alkyl-oder Cycloalkylresten substituiert ist, wie z.B. Toluol oder Xylol, halogenierte Kohlenwasserstoffe aus der Gruppe der Chlorbenzole oder C₁-C₁₀-Chloralkane, wie z.B. Chlorbenzol oder Methylenchlorid möglich. Die Lösungsmittel können Ether-, Nitril- und Esterfunktionen enthalten.

Besonders bevorzugt ist aus der Gruppe der aromatischen KW Toluol, aus der Gruppe der halogenierten KW Methylenchlorid, aus der Gruppe der Ether MTBE und Diethylether aus der Gruppe der Nitrile, Acetronitril und aus der Gruppe der Ester Ethylacetat als Lösungsmittel.

Es können auch Gemische der Lösungsmittel enthalten sein.

Als Reaktionsmedium wird ggf. mit Lösungsmittel verunreinigtes VC vorgelegt und darin CGC und tert.-Amin, bevorzugt Triethylamin, bei Temperaturen zwischen 30 und 60°C, bevorzugt zwischen 40 und 50°C zum Umsatz gebracht, bevorzugt wird ein Teil des durch Filtration isolierten Rohgemisches als Reaktionsmedium genutzt.

Üblicherweise wird bei der angegebenen Temperatur für einen Zeitraum von 2 bis 80, beorzugt 4 bis 40, besonders bevorzugt 5 bis 15 Std. gerührt.

Die Reihenfolge der Dosierung der Komponenten ist prinzipiell beliebig, bevorzugt wird Amin in VC vorgelegt und das CGC zu dosiert, besonders bevorzugt wird CGC und das Amin simultan dosiert.

Vorteil des Verfahrens ist neben der guten Ausbeute, dass der Filterkuchen der Filtration des Niederschlages an Ammoniumchlorid keinen großen Filtrationswiderstand aufbaut.

Das durch Filtration isolierte Ammoniumchloridsalz wird durch Wäsche mit einem inerten Lösungsmittel von anhaftendem Reaktionsgemisch befreit. Rohes Reaktionsgemisch und Waschfiltrat werden einer Vakuumdestillation zugeführt.

Die radikalische Polymerisation von VC kann durch den Zusatz von Radikalfängern, z.B. BHT, unterdrückt werden.

Im Folgenden wird das erfindungsgemäße Verfahren anhand einiger Beispiele illustriert, wobei die Beispiele jedoch nicht als Einschränkung des Erfindungsgedankens zu verstehen sind.

### Beispiele:

### Beispiel 1

In einen thermostatisierten 1L Planschlifftopf wurden 150 g Vinylencarbonat (ca. 99%ig), 31,2 g Triethylamin (99%ig) und 2 g BHT vorgelegt und auf 39°C thermostatisiert, anschließend wurden 283,5g Triethylamin und 310 g Chlorethylenglykolcarbonat (98%ig) binnen 3 h dosiert und dabei die Innentemperatur durch Kühlen unter 45°C gehalten. Worauf bei 41 bis 38°C Innentemperatur 4 h nachgerührt wurde.

Dann wurde über eine Filternutsche bei Raumtemperatur unter Vakuum abfiltriert.

Man erhielt 283g eines 82%igen rohen VC Filtrates.

Anschließend wurde der Filterkuchen 2 mal mit je 150 ml MTBE gewaschen.

Das 1. Waschfiltrat enthielt laut GC-Analyse 70 g VC.

Das 2. Waschfiltrat enthielt laut GC-Analyse 4,3 g VC.

Zusammen ergab dies eine Ausbeute von 73% VC, vorgelegtes VC abgezogen.

### Beispiel 2

In einen thermostatisierten 1L Planschlifftopf wurden 275 g Vinylencarbonat (ca. 82%ig) und 2 g BHT vorgelegt und auf 37°C thermostatisiert, anschließend werden 360 g Triethylamin (99%ig) und 400 g Chlorethylenglykolcarbonat (98%ig) in 3 h zudosiert und dabei die Innentemperatur unter 45°C gehalten, gefolgt von einer 4 h Nachrührphase bei 39°C.

Nach dem Abfiltrieren der Salze bei Raumtemperatur erhielt man 369g eines 81%igen rohen VC Filtrates.

Anschließend wurde der Filterkuchen 2 mal mit je 150 ml Toluol gewaschen.

Das 1. Waschfiltrat enthielt laut GC-Analyse 42 g VC.

Das 2. Waschfiltrat enthielt laut GC-Analyse 5,2 g VC.

Zusammen ergab dies eine Ausbeute von 74% VC, vorgelegtes VC abgezogen.

Die Filtration der Ammoniumchloridniederschläge wurde durch Verbackungen kaum behindert. Die Reaktoren wiesen kaum Ablagerungen auf!

### Beispiel 3 (Vergleichsbeispiel)

In einen thermostatisierten 1L Planschlifftopf wurden 150 g MTBE, 31,2 g Triethylamin (99%ig) und 2 g BHT vorgelegt und auf 39°C thermostatisiert, anschließend wurden 283,5g Triethylamin und 310 g Chlorethylenglykolcarbonat (98%ig) binnen 3 h dosiert und dabei die Innentemperatur durch Kühlen unter 45°C gehalten. Worauf bei ca.44°C Innentemperatur 4 h nachgerührt wurde.

Dann wurde über eine Filternutsche bei Raumtemperatur abfiltriert und mit 100ml MTBE nachgewaschen.

Die vereinigten Flüssigkeiten wurden destilliert. Man erhielt bei 55-62°C einen produktfreien Vorlauf von 485g.

Anschließend wurde bei 20 mbar und 66-84°C 92g eines 95%igen rohen Vinylencarbonats gewonnen, was einer Ausbeute von 40,3%d.Th. entsprach.

### Beispiel 4 (Vergleichsbeispiel)

In einen thermostatisierten 1L Planschlifftopf wurden 150 g MTBE, 31,2 g Triethylamin (99%ig) und 2 g BHT vorgelegt und auf 39°C thermostatisiert, anschließend wurden 283,5g Triethylamin und 310 g Chlorethylenglykolcarbonat (98%ig) binnen 3 h dosiert und dabei die Innentemperatur durch Kühlen unter 45°C gehalten. Worauf bei ca.42°C Innentemperatur 20 h nachgerührt wurde.

Dann wurde über eine Filternutsche bei Raumtemperatur abfiltriert und mit 100ml MTBE nachgewaschen.

Die vereinigten Flüssigkeiten wogen 490 g und wurden destilliert. Man erhielt bei 55-62°C einen produktfreien Vorlauf von 280g.

Anschließend erhielt man bei 20 mbar und 59-65°C 118g eines 97,6%ig rohen Vinylencarbonates, was einer Ausbeute von 52,9 % d.Th. entsprach.

Zurück blieben 92 g eines schwarzen Teeres.

Beide Vergleichsversuche zeigten starke Anbackungen an Gefäßwand und Rührer.

### Liste der Versuche zum Vinylencarbonat

| | |
|---|---|
| LRA | 6055 |
| LRA | 6058 |
| LRA | 6047 |
| LRA | 6048 |
| LRA | 6049 |
| LRA | 6046 |
| LRA | 7730 |
| LRA | 7733 |

## Patentansprüche

1. Ein Verfahren zur Herstellung von Vinylencarbonat durch Abspaltung von Chlorwasserstoff aus Chlorethylenglykolcarbonat (CGC) mit tertiären Aminen, **dadurch gekennzeichnet, dass** die Reaktion zwischen 30 und 60°C, in Vinylencarbonat als Reaktionsmedium, welches gegebenenfalls noch bis zu 10 Gew.-% weiteres Lösungsmittel, bezogen auf das Reaktionsmedium, enthält, durchgeführt wird.

2. Verfahren nach Anspruch 1, **dadurch gekennzeichnet, dass** durch Filtration isoliertes Rohgemisch als Reaktionsmedium genutzt wird.

3. Verfahren nach Anspruch 1 oder 2, **dadurch gekennzeichnet, dass** das durch Filtration isolierte Ammoniumchloridsalz durch Wäsche mit einem Lösungsmittel von anhaftenden Reaktionsgemisch befreit wird.

4. Verfahren nach einem der Ansprüche 1 bis 3, **dadurch gekennzeichnet, dass** bei Temperaturen zwischen 40 und 50°C die Reaktion durchgeführt wird.

5. Verfahren nach einem der Ansprüche 1 bis 5, **dadurch gekennzeichnet, dass** das weitere Lösungsmittel MTBE, Methylenchlorid, Toluol oder Acetonitril ist.

## Claims

1. Process for the production of vinylene carbonate by elimination of hydrogen chloride from chloroethylene glycol carbonate (CGC) with tertiary amines, **characterized in that** the reaction is carried out at between 30 and 60°C in vinylene carbonate as reaction medium, which optionally also contains up to 10% by weight of further solvent, based on the reaction medium.

2. Process according to Claim 1, **characterized in that** crude mixture isolated by filtration is used as the reaction medium.

3. Process according to Claim 1 or 2, **characterized in that** the ammonium chloride salt isolated by filtration is freed from adhering reaction mixture by washing with a solvent.

4. Process according to any of Claims 1 to 3, **characterized in that** the reaction is carried out at temperatures between 40 and 50°C.

5. Process according to any of Claims 1 to 5, **characterized in that** the further solvent is MTBE, methylene chloride, toluene or acetonitrile.

## Revendications

1. Procédé pour la préparation de carbonate de vinylène par élimination de chlorure d'hydrogène à partir de carbonate de chloréthylèneglycol (CGC) à l'aide d'amines tertiaires, **caractérisé en ce qu'**on effectue la réaction entre 30 et 60 °C, dans du carbonate de vinylène en tant que milieu réactionnel, qui éventuellement contient encore jusqu'à 10 % en poids d'un autre solvant, par rapport au milieu réactionnel.

2. Procédé selon la revendication 1, **caractérisé en ce qu'**on utilise comme milieu réactionnel du mélange brut isolé par filtration.

3. Procédé selon la revendication 1 ou 2, **caractérisé en ce qu'**on libère du mélange réactionnel adhérant, par lavage avec un solvant, le sel chlorure d'ammonium isolé par filtration.

4. Procédé selon l'une quelconque des revendications 1 à 3, **caractérisé en ce qu'**on effectue la réaction à des températures comprises entre 40 et 50 °C.

5. Procédé selon l'une quelconque des revendications 1 à 5, **caractérisé en ce que** l'autre solvant est le MTBE, le chlorure de méthylène, le toluène ou l'acétonitrile.
